# EUROPEAN PATENT APPLICATION

(11) **EP 4 374 859 A1**
(43) Date of publication of application: **29.05.2024**
(21) Application number: 22846286.7
(22) Date of filing: 22.07.2022
(51) Int. Cl.: A61K 31/454, A61P 17/00, A61P 11/00

(54) **PHARMACEUTICAL COMPOSITION FOR PREVENTION OR TREATMENT OF SYSTEMIC SCLEROSIS**

(30) Priority: 23.07.2021 KR 20210097163
(71) Applicant: Daewoong Pharmaceutical Co., Ltd., Hwaseong-si, Gyenoggi-do 18623 (KR)
(72) Inventor: LEE, Caroline Hee, Seoul 06170 (KR); BAE, Da Jeong, Seoul 06170 (KR); CHO, Min Jae, Seoul 06170 (KR); PARK, Joon Seok, Seoul 06170 (KR)
(74) Representative: Germain Maureau
(86) International application number: PCT/KR2022/010751
(87) International publication number: WO 2023/003416

(57) **Abstract**

The pharmaceutical composition according to the present invention can be usefully used for the prevention or treatment of systemic fibrosis.

## Description

### [TECHNICAL FIELD]

The present invention relates to a pharmaceutical composition for preventing or treating systemic sclerosis.

### [BACKGROUND ART]

Systemic sclerosis (SSc) is a degenerative disease that causes hyperplasia (thickening) or hardening (stiffening) of the skin, blood vessels, and internal organs, and belongs to an autoimmune rheumatic disease. The disease is chronic and can progress over a long period of time. Although the cause of systemic sclerosis has not yet been clarified, there are reports that certain chemical substances (toluene, benzene, vinyl chloride, silica, etc.) are also associated with the onset, and it is comprehensively diagnosed through the medical history, physical examination, histological tissue examination, and blood test. The degree of damage inside the organ can be confirmed by upper and lower gastrointestinal examinations, X-ray examination, lung function examination, electrocardiogram, and electrocardiographic examination, and the like.

Systemic sclerosis is generally not bad in the prognosis, but can be life-threatening if it spreads to the lungs, kidneys, or heart. Systemic sclerosis progresses to interstitial lung disease and pulmonary arterial hypertension, which may cause respiratory distress, and renal crisis accompanied by severe hypertension.

Systemic sclerosis is a type of autoimmune disease caused by an error in the internal immune system. Systemic sclerosis is medically cared at the department of rheumatology to advance a medical treatment for improving various symptoms caused by autoimmune inhibition. Systemic sclerosis is broadly classified into limited or diffuse cutaneous systemic sclerosis according to the extent of skin invasion, but some patients with systemic sclerosis show systemic sclerosis-associated interstitial lung disease (SSc-ILD) along with immunomodulatory disorders. The systemic sclerosis-associated interstitial lung disease is more commonly observed primarily in diffuse systemic sclerosis. In addition, 25 to 30% of these patients develop progressive SSc-ILD, which is known to be the leading cause of death in patients with systemic sclerosis (Lancet, 2017; 390:1685-1699).

The systemic sclerosis-associated interstitial lung disease is a respiratory disease that occurs in patients with systemic sclerosis, and is medically cared at the department of rheumatology to advance a medical treatment for improving . The systemic sclerosis-associated interstitial lung disease is highly associated with early mortality in patients with systemic sclerosis. The result of a study in the United States showed that the mortality rate due to pulmonary fibrosis in patients with systemic sclerosis increased more than fivefold from 6% to 33% in approximately 20 years, and interstitial lung disease became the most frequent cause of systemic sclerosis-related death. EULAR search research also revealed that it affects the mortality rate of more than 35% of patients with systemic sclerosis. (Steen VD, Medsger TA. Changes in causes of death in systemic sclerosis, 1972-2002. Ann Rheum Dis. 2007;66:940-4)

In regard to the risk factors for the onset or progression of interstitial lung disease in patients with systemic sclerosis, the disease occurs when conditions for the onset of interstitial lung disease are met for various reasons that have not yet been clarified, such as presence of dcSSc (diffuse cutaneous systemic sclerosis), African-Americans, age at onset (older age), shorter disease duration, and absence of anti-Scl-70/anti-topoisomerase I and anti-centromere antibodies. However, none of these risk factors are absolute, and the induction of systemic sclerosis does not necessarily lead to systemic sclerosis-associated interstitial lung disease. Further, since all patients do not show respiratory symptoms in diagnosis, doctors and researchers are enthusiastic about early diagnosis and development of therapeutic agents through various methods (Respiratory Research volume 20, Article number: 13 (2019)).

Particularly, looking at recent studies using clinical samples, systemic sclerosis-associated interstitial lung disease is derived from patients with systemic sclerosis, but it shows significant differences in various cytokines in the blood and various biomarkers such as IGFBP-1, MMP-9, and H3.1, which is thus considered to show that the disease characteristics of the patients are different. These studies are currently ongoing in order to be utilized as a biomarker for diagnosing systemic sclerosis-associated interstitial lung diseases in the systemic sclerosis cohort. This is data that can be the basis for the two diseases to be seen as a closely related but independent classification. In addition, the fact that MMP-7 and MMP-9 are increased in the blood specifically in patients with systemic sclerosis-associated interstitial lung disease than in patients with systemic sclerosis not only may be evidence supporting the cause of the onset of pulmonary fibrosis, and the rapid and high mortality rate in patients with systemic sclerosis-associated interstitial lung diseases reaching one-third, but also is interpreted as the basis capable of distinguishing between systemic sclerosis-associated interstitial lung diseases clinically as well as pathologically. ((i) Clin Epigenetics. 2020 Aug 17;12(1):124, (ii) Sarcoidosis Vasc Diffuse Lung Dis. 2015 Sep 14; 32(3):228-36, (iii) European Respiratory Journal 2021 58: 2101560).

To date, systemic sclerosis has no therapy that can stop the progression of the disease, and some drugs can alleviate certain symptoms and reduce organ damage. Non-steroidal anti-inflammatory drugs (NSAIDs) help alleviate joint pain, and calcium channel blockers can alleviate symptoms of Raynaud's phenomenon, but they can also cause gastrointestinal problems. Corticosteroids can alleviate myositis symptoms, immunosuppressive agents can alleviate lung inflammation, and high blood pressure drugs can alleviate acute kidney damage and blood pressure rise, but there is a limit in that it is only an effect of alleviating some symptoms, and no overall symptom-alleviating drugs exist.

For systemic sclerosis-associated interstitial lung disease, OFEV^{®} (Nintedanib) and Actemra^{®} (Tocilizumab) have been approved as a therapeutic agent. Nintedanib is a therapeutic agent for idiopathic pulmonary fibrosis and is known to improve lung function in patients with pulmonary fibrosis. However, nintedanib is effective only in improving pulmonary fibrosis and fails to prove its therapeutic effect on skin sclerosis symptoms, and thus, it has already been prescribed only as an adjuvant treatment for improving lung function in patients with end-stage systemic sclerosis-associated interstitial lung disease accompanied by advanced interstitial lung disease. Tocilizumab is an immunosuppressive agent for the treatment of rheumatoid arthritis and is known to alleviate symptoms of rheumatoid disease. However, tocilizumab did not prove fibrosis alleviation/improvement, and thus is used as a preventative-level adjuvant treatment for patients with early-stage systemic sclerosis-associated interstitial lung disease in which severe inhibition has not yet occurred. In other words, neither of the approved drugs provides a fundamental therapeutic effect to patients with systemic sclerosis as well as pulmonary disease accompanying systemic sclerosis, and thus is not prescribed as a first-line treatment, and patients still have to rely on immunosuppressive agents having severe side effects such as cyclophosphamide and mycophenolate.

Meanwhile, PRS (prolyl-tRNA synthetase) is one of the aminoacyl-tRNA synthetase (ARS) family, and serves to activate amino acids for protein synthesis. That is, ARS performs a translational function to form aminoacyl adenylate (AA-AMP) and then transfer the activated amino acid to the third end of the corresponding tRNA. Because ARS plays a key role in protein synthesis, ARS inhibition inhibits the growth and growth of all cells. Therefore, ARS is recognized as a promising target for antibiotics or therapeutic agents for diseases that must suppress cell overexpression (Nature, 2013, 494: 121-125).

PRS is present in, or functions as, a multisynthetase complex (MSC) in the form of Glutamyl-Prolyl-tRNA Synthetase (EPRS). Particularly, among various MSCs, EPRS functions as a transcriptional silencer that suppresses the production of vascular endothelial growth factor A (VEGF A), which is a key factor in angiogenesis. In addition, it has been reported that EPRS is closely related with various solid cancers (Nat. Rev. Cancer, 2011, 11, 708-718).

Therefore, the present inventors intensively studied methods for preventing or treating systemic sclerosis, and confirmed that a specific PRS inhibitor, which will be described later, can be usefully used for the prevention or treatment of systemic sclerosis, thereby completing the present invention.

### [DETAILED DESCRIPTION OF THE INVENTION]

### [Technical Problem]

It is an object of the invention to provide a pharmaceutical composition that can be usefully used for the prevention or treatment of systemic sclerosis.

### [Technical Solution]

In order to achieve the above object, there is provided a pharmaceutical composition for preventing or treating systemic fibrosis as follows:
A pharmaceutical composition for preventing or treating systemic fibrosis, comprising a compound represented by the following Chemical Formula 1, or a pharmaceutically acceptable salt thereof.

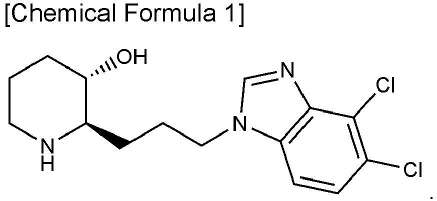

The compound represented by Chemical Formula 1, or a pharmaceutically acceptable salt thereof is a compound described in Korean Patent Registration No. 10-2084772, and specifically a substance described in Example 40 of the specification.

The pharmaceutical composition according to the present invention can be usefully used for the prevention or treatment of, especially, systemic sclerosis-associated interstitial lung disease (SSc-ILD) among other systemic sclerosis. In particular, the pharmaceutical composition according to the present invention has a dual effect of alleviating skin hardening of systemic sclerosis associated interstitial lung disease and improving lung function. The term "improvement of lung function" as used herein means that the ability to supply blood to the body, which is a substantial basic function of the lungs, is improved. This is not an effect that naturally follows from improvement of pulmonary fibrosis, and can be confirmed through oxygen saturation in the body separately from the pulmonary fibrosis index.

The term "prevention" as used herein refers to any act to delay or inhibit occurrence, spread or recurrence of the above diseases by administration of the composition of the present invention, and "treatment" refers to any act to improve or change the symptoms of the above diseases for the better by administration of the composition of the present invention.

Meanwhile, the compound represented by Chemical Formula 1 may be used in the form of a pharmaceutically acceptable salt. As the salt, an acid addition salt formed by a pharmaceutically acceptable free acid is useful. As the free acid, an inorganic acid and an organic acid may be used. Examples of the inorganic acid may include hydrochloric acid, bromic acid, sulfuric acid, phosphoric acid, and the like. Examples of the organic acid may include citric acid, acetic acid, lactic acid, maleic acid, gluconic acid, methanesulfonic acid, succinic acid, 4-toluene sulfonic acid, glutamic acid, aspartic acid or the like. Preferably, the pharmaceutically acceptable salt of the compounds represented by Chemical Formula 1 is hydrochloride.

Further, the compound represented by Chemical Formula 1 can be prepared in crystalline form or non-crystalline form. When the compound represented by Chemical Formula 1 is produced in crystalline form, it may be optionally hydrated or solvated. The present invention may include not only stoichiometric hydrates of the compound represented by Chemical Formula 1 but also compounds containing a various amount of water. The solvates of the compound represented by Chemical Formula 1 include both stoichiometric solvates and non-stoichiometric solvates.

The pharmaceutical composition according to the present invention can be formulated in types for oral or parenteral administrations according to a standard pharmaceutical practice. These formulations may contain additives such as pharmaceutically acceptable carrier, adjuvant or diluent in addition to the active ingredient.

Suitable carriers include, for example, physiological saline, polyethylene glycol, ethanol, vegetable oil, and isopropyl myristate and the like. Diluents include, for example, lactose, dextrose, sucrose, mannitol, sorbitol, cellulose and/or glycine and the like, but are not limited thereto. Further, the compounds of the present invention can be dissolved in oils, propylene glycol or other solvents commonly used in the preparation of injection solutions. Furthermore, the compounds of the present invention can be formulated in ointments or creams for topical application.

The pharmaceutical dosage forms of the compounds of the present invention can also be used in the form of a pharmaceutically acceptable salt or solvate thereof, and they can be used alone or in combination with other pharmaceutically active compounds, as well as in appropriate association. In one example, the pharmaceutical composition according to the present invention may further include other active ingredients used for the prevention or treatment of systemic fibrosis. Examples of such other active ingredients may include OFEV^{®} (Nintedanib) or Actemra^{®} (Tocilizumab). Additionally, when the pharmaceutical composition according to the present invention further comprises other active ingredient, the weight ratio of the compound represented by Chemical Formula 1, or a pharmaceutically acceptable salt thereof, and the other active ingredient is preferably 1:0.1 to 1:10.

The compounds of the present invention can be formulated into injections by dissolving, suspending or emulsifying the compounds in aqueous solvents such as common physiological saline or 5% dextrin, or in non-aqueous solvents such as synthetic fatty acid glycerides, higher fatty acid esters or propylene glycol. The formulation of the present invention may include conventional additives such as solubilizers, isotonic agents, suspending agents, emulsifying agents, stabilizers and preservatives.

The pharmaceutical composition according to the present invention may be administered via oral or parenteral routes. Depending on the method of administration, the composition according to the present invention may contain 0.001 to 99% by weight, preferably 0.01 to 60% by weight of the compound represented by Chemical Formula 1, or a pharmaceutically acceptable salt thereof.

The pharmaceutical composition according to the present invention may be administered to mammals such as a rat, a mouse, a domestic animal, a human, through various routes. The administration may be carried out through all possible methods, for example, oral, rectal, intravenous, intramuscular, subcutaneous, intra-endometrial, intracerebroventricular injection.

### [Advantageous Effects]

As described above, the pharmaceutical composition according to the present invention can be usefully used for the prevention or treatment of systemic fibrosis.

### [BRIEF DESCRIPTION OF THE DRAWINGS]

FIG. 1 shows the results of confirming the thickness of 3D skin organoid through H&E immunochemical staining in Experimental Example 1 of the present invention.
FIG. 2 shows the results of confirming the skin thickness of the mouse model of systemic scleroderma skin organoid transplantation through immunochemical staining in Experimental Example 2 of the present invention.
FIG. 3 shows the results of confirming the fibrosis factors Collagen I, Collagen III, and αSMA of the skin tissue of the mouse model of systemic scleroderma skin organoid transplantation through immunochemical staining in Experimental Example 2 of the present invention.
FIG. 4 shows an experimental schedule for mice in Experimental Example 3 of the present invention.
FIG. 5 shows the results of changes in the weight of the mouse in Experimental Example 3-1 of the present invention.
FIG. 6 shows the results of changes in lung weight of mice in Experimental Example 3-2 of the present invention.
FIG. 7 shows the results of evaluation of lung function of mice in Experimental Example 3-3 of the present invention.
FIG. 8 shows the results of evaluation of the skin thickness of mice in Experimental Example 3-4 of the present invention.
FIG. 9 shows the results of measuring the collagen content in the body of mice in Experimental Examples 3-5 of the present invention.

### [DETAILED DESCRIPTION OF THE EMBODIMENTS]

Below, the present invention will be described in more detail by way of examples. However, these examples are provided for illustrative purposes only, and should not be construed as limiting the scope of the present invention to these examples.

### EXAMPLE

The following compound was prepared in the same manner as in Example 40 of Korean Patent Registration No. 10-2084772, and was hereinafter referred to as 'active ingredient' or 'Example'.

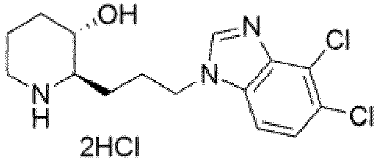

¹H NMR (500 MHz, MeOD): δ 9.67 (s, 1H). 8.02 (d, 1H), 7.82 (d, 1H), 4.62 (m, 2H), 3.60 (m, 1H), 3.28 (m, 1H), 2.99 (m, 2H), 2.25 (m, 2H), 2.08 (m, 2H), 1.99 (m, 1H), 1.78 (m, 2H), 1.54 (m, 1H)

### Experimental Example 1: Confirmation of skin hardening alleviation effect in 3D skin organoid model

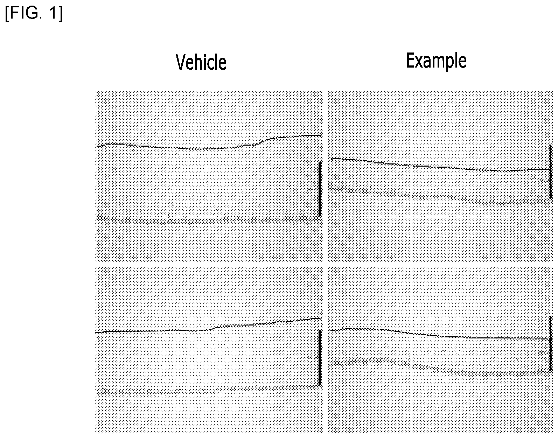

A 3D skin organoid was prepared using iPSC-derived fibroblasts from patients with systemic dermatosis according to the method described in the literature (Lancet. 2009 Nov 21;374(9703):1745-53). When 1 uM of active ingredient was treated with DMSO solution, it was confirmed through H&E immunochemical staining that the thickness of the 3D skin organoid was reduced (FIG. 1). The control group was treated with only DMSO.

### Experimental Example 2: Confirmation of skin hardening alleviation effect in 3D skin organoid transplantation mouse model

The previously constructed 3D skin organoid derived from the systemic scleroderma patient iPSC was transplanted into SCID mice to construct a mouse model of systemic scleroderma. The mouse skin tissue was cut into 1 × 2 cm, and then 3D skin organoid skin tissue was sutured to the mouse skin. For a skin transplantation method, it was transplanted to mice or the like using a tie-over dressing method used clinically. After placing a gauze on the suture site, it was tied with a suture and dressed with a band.

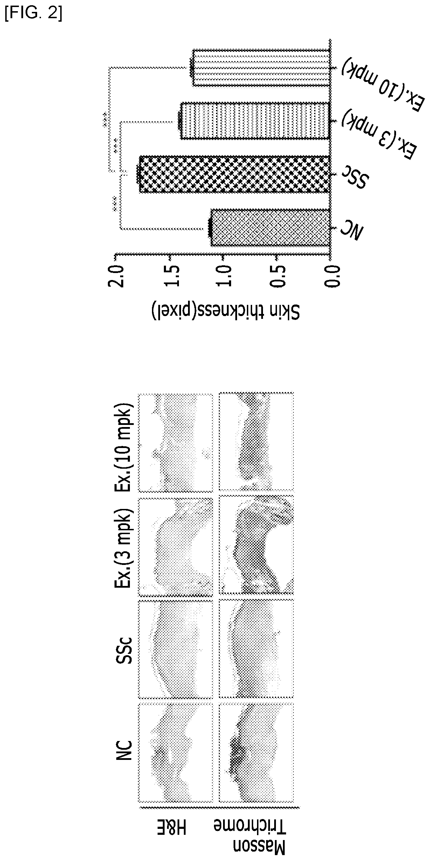

The active ingredient was subcutaneously administered at a dose of 3 mg/kg and 10 mg/kg daily for 2 weeks to a mouse model of systemic scleroderma skin organoid transplantation. For the negative control group (NC), a physiological saline was administered to a normal skin tissue, and for the positive control group (SSc), a physiological saline was administered to a systemic scleroderma skin organoid transplanted skin tissue. The skin thickness, which is the primary evaluation index of systemic scleroderma, was measured through immunochemical staining, and the results are shown in FIG. 2. As shown in FIG. 2, it was confirmed that the thickness of the skin tissue was significantly reduced by the active ingredient (***p<0.001).

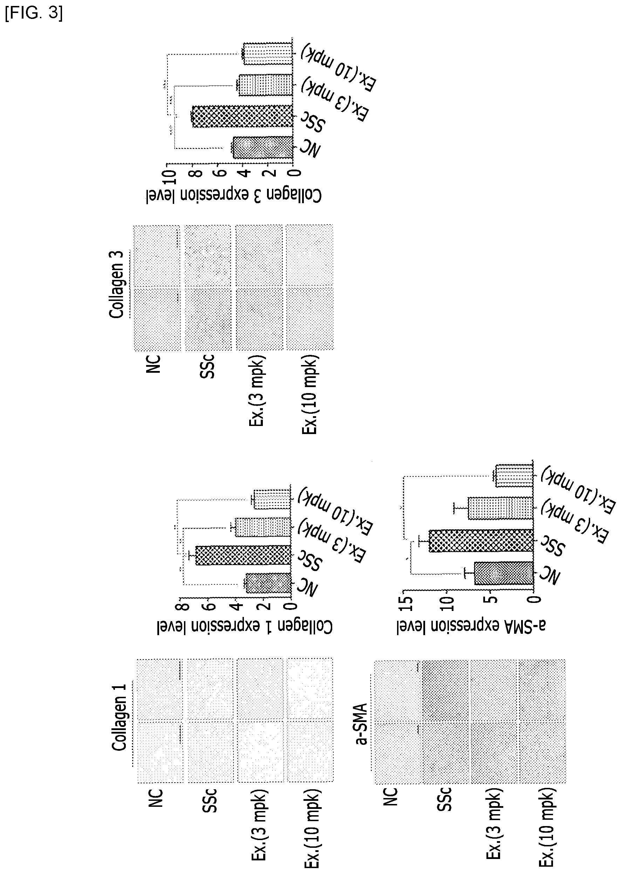

To confirm whether the fibrosis factor is reduced in systemic scleroderma skin tissue by the active ingredient, immunochemical staining was performed and the results are shown in FIG. 3. As shown in FIG. 3, it was confirmed that the fibrotic factors, that is, Collagen I, Collagen III, and αSMA, were significantly reduced by the active ingredient (*p<0.05, ***p<0.001).

### Experimental Example 3: Confirmation of therapeutic effect in systemic sclerosis- associated interstitial lung disease (SSc-ILD)

Seven-week-old C57BL/6 mice were obtained and acclimatized indoors for 5 days or more. For the systemic sclerosis-related interstitial lung disease model, an osmotic pump injection model was designed. For that reason, if it is not modeling using an osmotic pump, it is a model in which BLM should be injected subcutaneously into the back of the test animal every day for 4 weeks, and this is because these tests can cause very extreme pain at the site of administration.

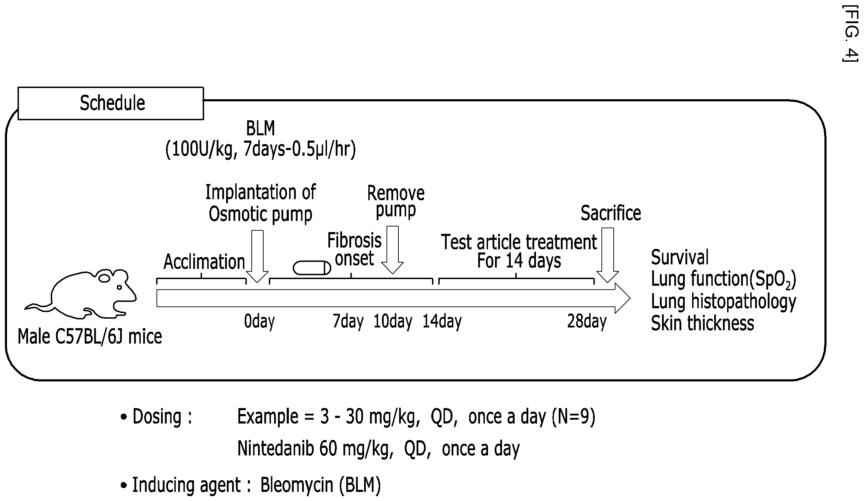

As shown in FIG. 4, the drug administration rate using the osmotic pump was 0.5 ul/hr (7 days), and the size of the osmotic pump was 1.5 cm. The incision site was sutured, an osmotic pump was inserted, bleomycin was administered for 1 week, and then the osmotic pump was removed on the 10th day. It was confirmed that systemic sclerosis-associated interstitial lung disease was induced in a mouse model administered with BLM for 1 week, and specific experimental groups are shown in Table 1 below.

**[Table 1]**

| | Number of animals (head) | Administration route (Osmotic pump) | | | Administration route (oral) | | |
|---|---|---|---|---|---|---|---|
| Test gro up | | Administ ration substan ce | Administra tion dose (U/kg) | Administrat ion liquid dose (ul) | Administra tion substance | Administra tion dose (mg/kg) | Administra tion liquid dose (ul) |
| G1 | 9 | Saline | - | 100 | Saline | - | 100 |
| G2 | 9 | BLM | 100U/kg | 100 | Saline | - | 100 |
| G3 | 9 | BLM | 100U/kg | 100 | Nintedanib | 60 | 100 |
| G4 | 9 | BLM | 100U/kg | 100 | Active ingredient | 3 | 100 |
| G5 | 9 | BLM | 100U/kg | 100 | Active ingredient | 10 | 100 |
| G6 | 9 | BLM | 100U/kg | 100 | Active ingredient | 30 | 100 |

### Experimental Example 3-1: Body weight (%)

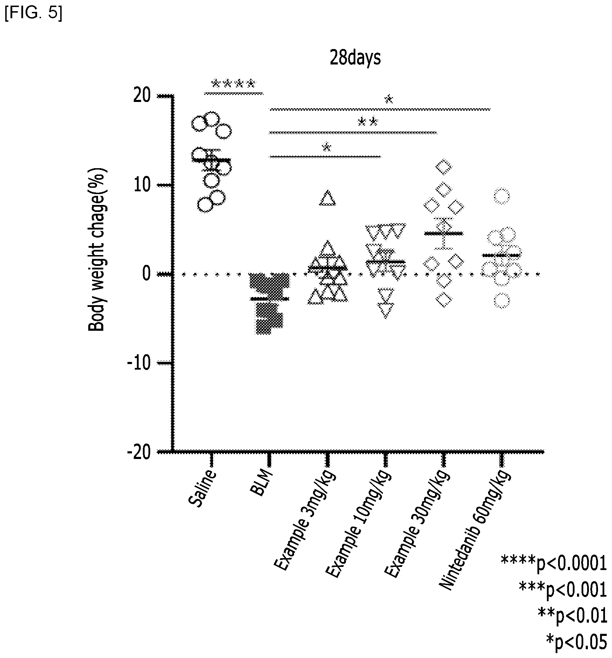

Based on the results of 28 days of administration of the active ingredient (Table 2 and FIG. 5), it was confirmed that changes in body weight showed significant results from 3 mg/kg of active ingredient, and at 30 mg/kg, the body weight was significantly increased to p<0.01 compared to the vehicle.

**[Table 2]**

| | Saline | BLM | Active ingredient (3 mg/kg) | Active ingredient (10 mg/kg) | Active ingredient (30 mg/kg) | NIN |
|---|---|---|---|---|---|---|
| 1 | 16.94 | -0.73 | 8.65 | 0.48 | 9.52 | 2.46 |
| 2 | 16.1 | -1.15 | 3.02 | 0.14 | 5.35 | 4.14 |
| 3 | 17.47 | -1.19 | -0.36 | 4.88 | -2.86 | 1.78 |
| 4 | 11.99 | -5.16 | 1.07 | 4.78 | 7.57 | 4.49 |
| 5 | 10.58 | -4.1 | -2.45 | 4.62 | 12.09 | -0.45 |
| 6 | 7.85 | -5.9 | -2.14 | 2.54 | 7.78 | 0.58 |
| 7 | 8.63 | -0.75 | 1.34 | 1.96 | 2.13 | 8.79 |
| 8 | 13.47 | -4 | -0.33 | -4.1 | 2.34 | 0.44 |
| 9 | 12.59 | -2.17 | -1.93 | -2.48 | 1.45 | -2.94 |

### Experimental Example 3-2: Lung weight

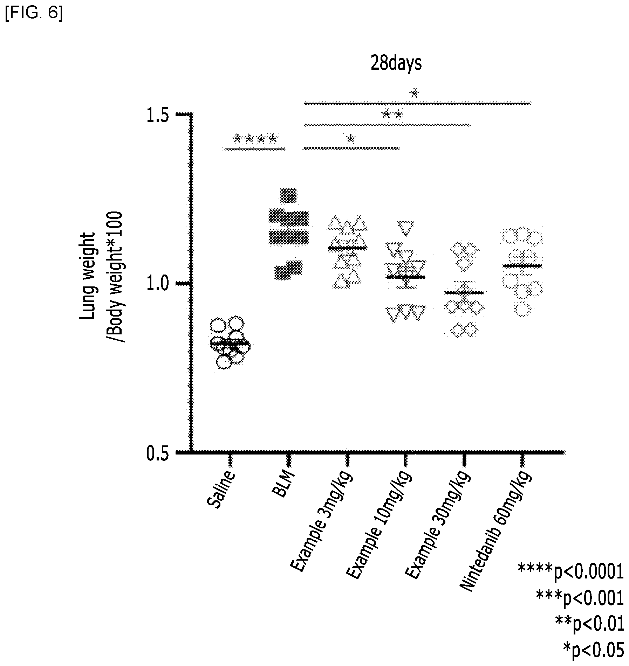

It was confirmed that the change in lung weight showed a significant tendency only at 30 mg/kg, but when corrected by body weight, it showed a significance from 10 mg/kg, and at 30 mg/kg, the significance increased to p<0.01 (Table 3 and FIG. 6).

**[Table 3]**

| | Saline | BLM | Active ingredient (3 mg/kg) | Active ingredient (10 mg/kg) | Active ingredient (30 mg/kg) | NIN |
|---|---|---|---|---|---|---|
| 1 | 0.8 | 1.03 | 1.14 | 1.16 | 0.94 | 0.99 |
| 2 | 0.8 | 1.05 | 0.98 | 0.99 | 0.98 | 0.98 |
| 3 | 0.8 | 1.14 | 1.15 | 0.93 | 1.09 | 1.16 |
| 4 | 0.8 | 1.19 | 0.92 | 1.08 | 0.97 | 0.89 |
| 5 | 0.8 | 1.19 | 1.01 | 0.98 | 1.10 | 0.96 |
| 6 | 0.8 | 1.14 | 1.14 | 1.22 | 0.99 | 0.99 |
| 7 | 0.8 | 1.26 | 1.08 | 1.14 | 0.90 | 1.04 |
| 8 | 0.9 | 1.20 | 0.98 | 1.12 | 0.98 | 0.95 |
| 9 | 0.9 | 1.14 | 1.08 | 0.95 | 1.03 | 1.01 |

### Experimental Example 3-3: Lung Function Evaluation (SpO₂ (%))

This lung function evaluation is an experiment to show the therapeutic effect of this active substance in patients with systemic sclerosis-associated interstitial lung diseases, and is the most direct and important evaluation index that has the greatest influence on the symptoms and quality of life of patients with systemic sclerosis-associated interstitial lung diseases, unlike the general pulmonary fibrosis ameliorating effect. This is an experiment that can most directly show the effect of improving lung function in an animal model of systemic sclerosis-associated interstitial lung disease by measuring the oxygen concentration in the body.

On the 21st day, it was measured with a SpO₂ measuring device (Berry, Veterinary Pulse Oximeter) via the abdominal side of the mouse, and the results are shown in Table 5 and FIG. 3.

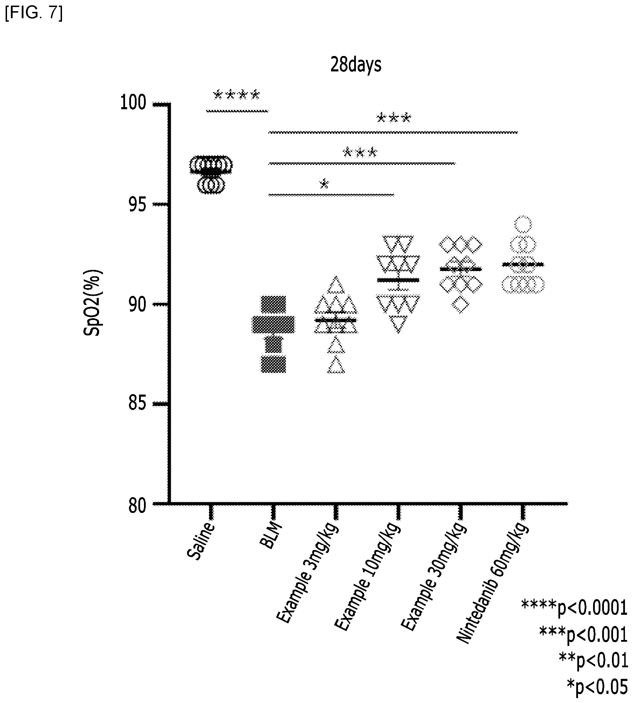

As shown in FIG. 7, significant results were obtained from 10 mg/kg of the active ingredient, and 30 mg/kg of the active ingredient exhibited a higher oxygen saturation (p<0.001) as compared with the vehicle. In addition, it was confirmed that the active ingredient (30 mg/kg ) administration group exhibited an oxygen saturation recovery rate, comparable to that of nintedanib (60 mg/kg) administration group which is a control group.

### Experimental Example 3-4: Evaluation of skin thickness

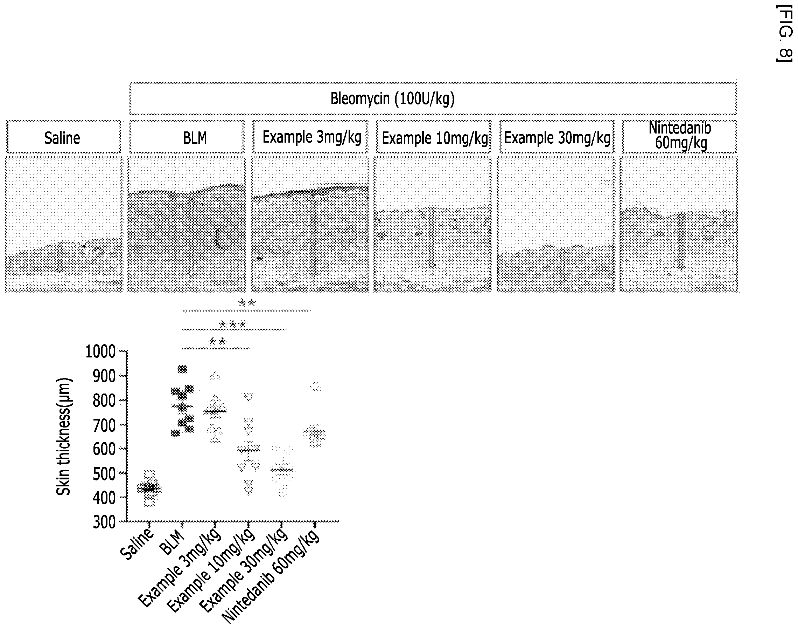

On the day 28, skin tissues were extracted from mice and subjected to H&E and Mansson's Trichrome (MT stain). The skin thickness of 10 sites was measured randomly through the image of the photograph in which stain progressed (progressed at a minimum interval of 200 ~300 um). The entire area of the photograph was measured as evenly as possible, and the areas where the stain tissue was damaged were excluded from the measurement. The results are shown in FIG. 8 and Table 4.

As shown in FIG. 8, it was confirmed that in the BLM group, the collage was well filled and induced even in the area where adipose should be placed in addition to the dermis of the skin. It was confirmed that significant results were obtained from 10 mg/kg of active ingredient, and 30 mg/kg of active ingredient exhibited very significant results (P<0.0001 or less). In addition, when the active ingredient was administered as a whole, the collagen density in the skin was decreased through the low degree of MT stain staining. In particular, it was confirmed that skin thickness did not significantly decrease at 3 mg/kg of active ingredient, but the density of collagen in the skin showed the tendency to decrease. In particular, it was confirmed that 10 mg/kg of the active ingredient exhibited excellent skin thickness improvement effect superior as compared with 60 mg/kg of nintedanib.

**[Table 4]**

| µm | Saline | BLM | Active ingredient (3 mg/kg) | Active ingredient (10 mg/kg) | Active ingredient (30 mg/kg) | NIN |
|---|---|---|---|---|---|---|
| 1 | 436.567 | 663.945 | 677.319 | 593.826 | 443.336 | 620.302 |
| 2 | 412.602 | 723.118 | 744.986 | 601.558 | 601.558 | 651.572 |
| 3 | 445.578 | 844.957 | 904.141 | 809.106 | 558.530 | 856.355 |
| 4 | 437.536 | 819.822 | 641.841 | 708.308 | 479.961 | 664.973 |
| 5 | 378.447 | 835.348 | 809.254 | 526.170 | 475.515 | 675.558 |
| 6 | 494.174 | 926.507 | 771.562 | 521.027 | 412.271 | 623.019 |
| 7 | 458.348 | 706.216 | 778.660 | 425.415 | 525.311 | 651.831 |
| 8 | 420.379 | 681.761 | 688.725 | 456.862 | 593.963 | 660.809 |
| 9 | 443.032 | 768.854 | 759.037 | 672.616 | 526.388 | 649.479 |

### Experimental Example 3-6: Measurement of Collagen Content in the Body

An attempt was made to measuring the amount of hydroxyproline in the mouse and thus indirectly measure the collagen content. Analysis was performed using INSOLUBLE Collagen Assay (Biocolor, S2000), and the details are as follows.

First, 10 mg of frozen skin tissue was pulverized with 100 ul of fragmentation reagent. 100 ul of 37% HCl was added and incubated at 65°C for 3 hours. The contents of the tube were shaken at 30 minute intervals to aid tissue disintegration. Centrifugation was performed at 12,000 rpm for 10 minutes. After centrifugation, it was transferred to a 1.5 ml tube. 50 ul was taken from a sample between 10 and 100 ul, distilled water was added, and the sample was prepared so as to match with 100 ul.

1 ml of dye was added to 100 ul of the prepared sample, and mixed for 30 minutes. The mixture was centrifuged at 12,000 rpm for 10 minutes, and then transferred to a new tube. The dye was removed with 750 ul of ice-cold acid-salt wash reagent. After centrifugation at 12,000 rpm for 10 minutes, it was transferred to a new tube. The tube was mixed with the collagen-binding dye using a vortex mixer, and gollagen binding dye was dissolved within 10 minutes to complete the measurement preparation (measurement should be completed within 2-3 hours). The tube cap was closed until it was ready to measure absorbance.

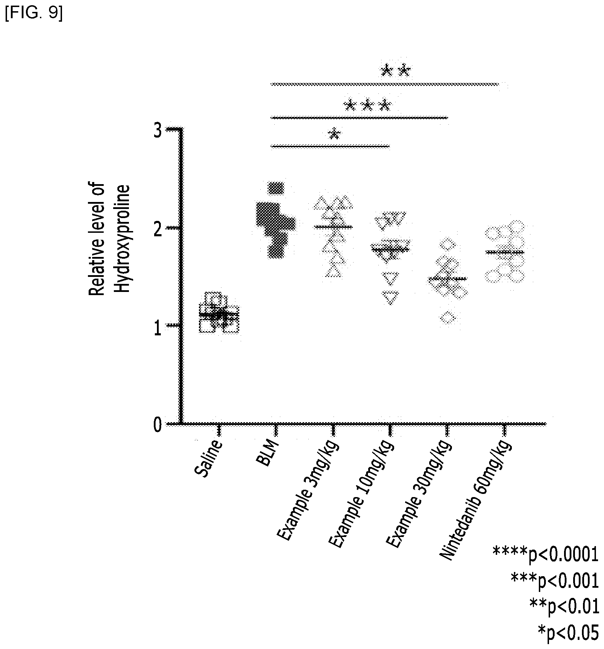

200 ul of each sample was transferred to an individual well of a 96 micro-well plate, and the absorbance was measured at 560 nm. The hydroxyproline value was represented by the ratio value compared to a normal group, and the results are shown in Table 5 and FIG. 9.

As a result of confirming the BLM group, it was confirmed that the amount of hydroxyproline in the skin was increased twice or more as compared with the saline group, and thus, the modeling was performed well. In addition, significant results were shown from 10 mg/kg of active ingredient, and 30 mg/kg of active ingredient showed a very significant result (P<0.001 or less).

**[Table 5]**

| | Saline | BLM | Active ingredient (3 mg/kg) | Active ingredient (10 mg/kg) | Active ingredient (30 mg/kg) | NIN |
|---|---|---|---|---|---|---|
| 1 | 1.00 | 2.19 | 1.74 | 1.57 | 1.75 | 1.37 |
| 2 | 1.24 | 2.08 | 1.51 | 2.16 | 1.77 | 1.43 |
| 3 | 1.28 | 2.20 | 1.57 | 1.82 | 2.09 | 1.65 |
| 4 | 1.13 | 1.98 | 2.01 | 2.10 | 1.28 | 1.45 |
| 5 | 1.16 | 1.76 | 1.51 | 2.27 | 1.70 | 1.34 |
| 6 | 1.13 | 2.05 | 1.96 | 2.26 | 1.48 | 1.83 |
| 7 | 1.00 | 1.89 | 1.66 | 1.70 | 1.81 | 1.63 |
| 8 | 1.06 | 2.04 | 1.94 | 2.26 | 2.04 | 1.53 |
| 9 | 1.08 | 2.40 | 1.85 | 1.93 | 2.09 | 1.08 |

As can be confirmed through the above experimental results, the active ingredient of the present invention exhibited two completely different effects, that is, a therapeutic effect on systemic sclerosis and an improvement in lung function inhibition of systemic sclerosis-associated interstitial lung diseases. Such dual effects are an unpredictable part through any of the therapeutic effects, and is understood to be a characteristic of the active substance of the present invention.

## Claims

1. A pharmaceutical composition for preventing or treating systemic fibrosis, comprising a compound represented by the following Chemical Formula 1, or a pharmaceutically acceptable salt thereof.

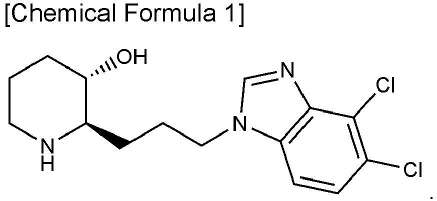

2. The pharmaceutical composition according to claim 1, wherein:
the systemic fibrosis is systemic sclerosis-associated interstitial lung disease (SSc-ILD).

3. The pharmaceutical composition according to claim 2, wherein:
the pharmaceutical composition has a dual effect of alleviating skin hardening of systemic sclerosis-associated interstitial lung diseases and improving lung function.

4. The pharmaceutical composition according to claim 1, wherein:
the pharmaceutically acceptable salt is hydrochloride.

5. The pharmaceutical composition according to claim 1, wherein:
the pharmaceutical composition further comprises other active ingredient used for the prevention or treatment of systemic fibrosis.

6. The pharmaceutical composition according to claim 5, wherein:
the other active ingredient used for the prevention or treatment of systemic fibrosis is Nintedanib or Tocilizumab.
